# EUROPEAN PATENT SPECIFICATION

(11) **EP 1 201 247 B1**
(45) Date of publication and mention of the grant of the patent: **27.01.2010**
(21) Application number: 01309157.4
(22) Date of filing: 29.10.2001
(51) Int. Cl.: A61K 38/20, A61P 35/00, A61K 31/549

(54) **Treatment of metastatic renal cell carcinoma**
Behandlung des metastasierenden Karzinoms der Niere
Traitement du carcinome rénal métastasé

(30) Priority: 27.10.2000 US 243409 P
(43) Date of publication of application: 02.05.2002
(60) Divisional application: 09009373.3
(73) Proprietor: Ed Geistlich Söhne AG Für Chemische Industrie, 6110 Wolhusen (CH)
(72) Inventor: Redmond, H. Paul, c/o Cork University Hospital, Cork (IE); Pfirrmann, Rolf W., 6006 Lucerne (CH)
(74) Representative: Pett, Christopher Phineas

(56) References cited:
- EP-A- 1 066 830
- WO-A-92/00743

## Description

The present invention relates to the field of treating tumor metastases and cancer.

Interleukin-2 (IL-2) is an agent which has been suggested for inhibiting tumor cell growth. However, administration of IL-2 to patients presents severe toxicity problems, since IL-2 elicits an extremely strong systemic inflammatory response syndrome (SIRS) reaction in patients. Toxicity of IL-2 is so severe that approximately 70% of patients cannot tolerate treatment.

Additionally, a common problem in patients undergoing cancer treatment is tumor recurrence or metastasis.

Thus, despite the advances in cancer treatment, there remains a significant need in the art for new and improved cancer treatment therapies.

In accordance with the present invention, tumor metastasis is inhibited in a cancer patient by administering to said patient a combination therapy comprising effective amounts of IL-2 and the methylol transfer agent taurolidine.

It has surprisingly been found that the methylol transfer agent taurolidine can reduce or substantially eliminate the severe toxicity and side effects of IL-2 in a combination therapy for inhibiting tumor metastases and treating cancer in patients, while it has unexpectedly been found that the efficacy of IL-2 is actually enhanced by the methylol transfer agent in the combination therapy of the present invention.

IL-2 when used in accordance with the present invention includes natural or recombinant Interleukin-2, or biologically active derivatives or substantial equivalents thereof.

Methylol transfer agents such as taurolidine and taurultam have been diclosed as anticancer agents in WO92/00743 and EP-A-1066830. Other anticancer methylol-containing compounds may be found among those identified in WO 01/39763.

The combination therapy of the present invention has been found to be particularly effective in inhibiting metastatic renal cell carcinoma.

Effective daily dosage amounts of IL-2 may comprise pharmaceutical dosage units within the range of 1,000,000-100,000,000 units (U) IL-2 per m² body surface area. Dosage amounts of IL-2 also may be found within the range of 100,000-1,000,000 U per kilogram body weight. Dosage amounts of IL-2 further may be found within the range of 0.1-100 micrograms IL-2 per kilogram body weight.

Effective dosage amounts of the methylol transfer agent taurolidine in accordance with the present invention may comprise pharmaceutical dosage units within the range of about 0.1-1,000 mg/kg. Preferred dosages may be in the range of about 10-20 grams per administration.

Pharmaceutical dosage units of the combined therapy of the present invention may be administered by any suitable route, which include oral, topical or peritoneal administration, e.g., subcutaneously, intraperitoneally, intramuscularly, or intravenously eg., by infusion or injection.

In preferred embodiments, 250 ml of taurolidine 2% solution is administered by intravenous infusion about 1-6 times per day, more preferably about 2-4 times per day, during a treatment period, concurrently with administration of about 10,000,000-40,000,000 units m² IL-2 by intravenous infusion per day during the treatment period.

The present invention thus is directed to use of a combination of IL-2 and the methylol transfer agent taurolidine, in the manufacture of a medicament for simultaneous, separate or sequential treatment of metastatic renal cell carcinoma.

The invention is further illustrated by the following non-limiting example.

### Example

A 50 year old patient diagnosed with metastatic renal cell carcinoma was treated as follows.

| | |
|---|---|
| Presentation | Haemoptysis - 2° to pulmonary metastases. Noted to have hepatic metastases, in addition to a large mass in the left kidney. |
| | |
| Treatment | IL-2 and Taurolidine |
| | |
| Regimen | Interleukin-2 |
| | |
| | Day 1: 18 million units/m² IL-2 infusion over 6 hours |
| | |
| | Day 2: 18 million units/m² IL-2 infusion over 12 hours |
| | |
| | Day 3: 18 million units/m² IL-2 infusion over 24 hours |
| | |
| | Days 4-7: 18 million units/m² IL-2 infusion over 78 hours |
| | |
| | Taurolidine |
| | |
| | Taurolidine 2% 250 ml infusion over two hours, twice daily during IL-2 administration |
| | |
| | Completed five courses of the above |
| | |
| Further treatment | Left radical nephrectomy |

After five years, the patient is alive and well, with no evidence of disease on imaging.

## Claims

1. Use of interleukin-2 and taurolidine in the manufacture of a combined preparation for simultaneously, separately or sequentially treating metastatic renal cell carcinoma.

2. Use as claimed in claim 1 wherein said combined preparation is manufactured such that said interleukin-2 and said taurolidine are each present as separate pharmaceutical dosage units.

## Patentansprüche

1. Verwendung von Interleukin-2 und Taurolidin bei der Herstellung einer kombinierten Zubereitung für die simultane, getrennte oder sequenzielle Behandlung von metastasierenden Nierenzellkarzinomen.

2. Verwendung nach Anspruch 1, bei der die kombinierte Zubereitung so hergestellt wird, daß das Interleukin-2 und das Taurolidin jeweils als getrennte pharmazeutische Dosierungseinheiten vorhanden sind.

## Revendications

1. Utilisation de l'interleukine 2 et de la taurolidine dans la fabrication d'une préparation combinée destinée à traiter simultanément, séparément ou séquentiellement un carcinome métastasé des cellules rénales.

2. Utilisation selon la revendication 1, où ladite préparation combinée est fabriquée de telle manière que ladite interleukine 2 et ladite taurolidine sont chacune présentes sous forme d'unités galéniques distinctes.
